(19) Europäisches Patentamt
European Patent Office
Office européen des brevets

(11) **EP 1 923 000 A2**

(12) **DEMANDE DE BREVET EUROPEEN**

(43) Date de publication:
**21.05.2008 Bulletin 2008/21**

(51) Int Cl.:
*A61B 6/00* (2006.01)     *G01R 33/58* (2006.01)
*G01T 1/00* (2006.01)

(21) Numéro de dépôt: **07370024.7**

(22) Date de dépôt: **15.11.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **15.11.2006 FR 0609994**

(71) Demandeurs:
• **Centre Hospitalier Regional Universitaire de Lille 59037 Lille Cédex (FR)**
• **UNIVERSITE DU DROIT ET DE LA SANTE (LILLE II) F-59800 Lille (FR)**

(72) Inventeurs:
• **Rousseau, Jean 37000 Tours (FR)**
• **Vermandel, Maximilien 59110 La Madeleine (FR)**
• **Huglo, Damien 59310 Nomain (FR)**

(74) Mandataire: **Matkowska, Franck 9 rue Jacques Prévert 59650 Villeneuve d'Ascq (FR)**

(54) **Fantôme pour le contrôle de qualité en imagerie tomographique, et notamment en imagerie TEP**

(57) Le fantôme (1) est destiné à être utilisé pour le contrôle de qualité en imagerie tomographique, et notamment en imagerie médicale du type TEP, TEMP, TDM, IRM. Il comporte un bac (2) et au moins deux inserts (4 ou 4' ou 4") fixés à l'intérieur du bac (2), chaque insert comportant un premier plan de calage (40a), un plan de détection (44a), qui est incliné par rapport audit premier plan de calage (40a) d'un angle ($\alpha$) inférieur à 90°, et au moins un deuxième plan de calage (41 a) qui est opposé au sommet de l'angle ($\alpha$) et qui est perpendiculaire au premier plan de calage (40a). Le bac (2) est de forme parallélépipédique ; chaque insert (4 ou 4' ou 4") est fixé à l'intérieur du bac (2) d'une part de telle sorte que l'un (40a ou 41a) des plans de calage de l'insert est parallèle à une première paroi du bac (2), et l'autre (41a ou 40a) plan de calage de l'insert est parallèle à une autre paroi du bac (2) perpendiculaire à ladite première paroi, et d'autre part de telle sorte que lesdits deuxièmes plans de calage (41 a) des inserts sont perpendiculaires.

Fig.1

**EP 1 923 000 A2**

**Description**

Domaine technique

La présente invention concerne le domaine du contrôle de qualité en imagerie tomographique 3D, et trouve préférentiellement, mais pas exclusivement son application au contrôle de qualité en imagerie TEP (Tomographie par Emission de Positons). Dans ce domaine, l'invention a pour objet un nouveau fantôme qui permet, par tomographie au moyen d'un appareil d'imagerie, et en association avec un programme de calcul, de déterminer un ou plusieurs paramètres caractérisant la qualité des images tomographiques acquises au moyen dudit appareil d'imagerie, tels que notamment la résolution spatiale selon différentes direction, le rapport signal/bruit, le profil de coupe ou l'uniformité. L'invention trouve principalement son application dans le domaine du contrôle de qualité d'un appareil d'imagerie médicale.

Art antérieur

Dans le domaine de l'imagerie médicale 3D, différentes technologies d'acquisition d'image permettant une tomographie du corps humain on été développées, en vue du diagnostic notamment de tumeurs cancéreuses ou précancéreuses. Parmi ces différentes technologies, on peut citer par exemple, les appareils d'imagerie fonctionnant par résonance magnétique (IRM), les appareils d'imagerie permettant une tomographie par émission monophotonique (TEMP), les appareils d'imagerie permettant une tomographie par émission de positons (TEP), la tomodensitométrie à rayons X (TDM).
Chaque type d'imagerie (IRM, TEMP, TEP, TDM...) a ses propres avantages et inconvénients, et il est préférable de choisir celle la plus adaptée au diagnostique médical recherché.
Quel que soit le type d'appareil d'imagerie par tomographie utilisé, il est important que le fabricant puisse, avant de livrer un appareil à un client, contrôler la qualité des images tomographiques acquises par l'appareil. Il est ensuite également important pour l'utilisateur d'un appareil d'imagerie médical de pouvoir périodiquement contrôler dans le temps la qualité des images tomographiques acquises par l'appareil.
Différents dispositifs de contrôle, communément appelés fantômes, sont commercialisés à ce jour. D'une manière générale, pour le contrôle de qualité, on réalise par tomographie des acquisitions du fantôme, au moyen de l'appareil d'imagerie médicale que l'on souhaite contrôler, et l'analyse de qualité est effectuée sur les données natives ou sur les coupes reconstruites, le cas échéant en calculant automatiquement au moyen d'un programme de calcul adapté différents paramètres de qualité.
Différentes structures connues de fantômes, sont décrites par exemple dans les publications suivantes : demande de brevet européen EP 0 082 733, brevet américain US 7 056 019, demande de brevet américain US 2005/0008126.
Plus particulièrement, dans le domaine de l'imagerie TEP, il existe à ce jour principalement deux protocoles de contrôle qualité reconnus internationalement : le protocole NEMA (National Electrical Manufacturers Association), dont la version la plus récente est référencée NEMA NU 2:2001, et le protocole IEC NE 61675-1 :1998 par IEC (International Electrotechnical Commission).
Ces deux protocoles définissent des recommandations, des méthodes d'acquisition d'image et des algorithmes de calcul, qui doivent être mis en oeuvre pour obtenir différents paramètres de qualité d'image prédéfinis, tel que notamment la résolution spatiale, l'uniformité, ...
Sur la base des recommandations de ces deux protocoles, différents fantômes, plus ou moins sophistiqués, sont commercialisés à ce jour.
Cependant, l'utilisation de ces fantômes, conformément aux recommandations de l'un ou l'autre des deux protocoles précités, implique des opérations de contrôle longues et fastidieuses, en termes de manipulation du fantôme, d'acquisition et de traitement d'image, et d'analyse des résultats. Il en résulte en pratique que ces opérations de contrôle, notamment en imagerie TEP, sont le plus souvent réalisées par le fabricant de l'appareil, afin de vérifier, avant livraison à l'utilisateur final, la conformité de l'appareil d'imagerie médicale, et sont ensuite le plus souvent négligées par l'utilisateur de l'appareil d'imagerie, ou dans le meilleur des cas sont effectuées de manière très sporadique.
On a également déjà proposé dans la demande de brevet GB-A-2 155 187 un fantôme pour le contrôle de qualité d'un appareil d'imagerie fonctionnant par résonance magnétique (IRM). Ce fantôme comporte un bac de forme cylindrique, fermé par un couvercle, et deux inserts présentant une section transversale en forme de triangle rectangle. Chaque insert présente un plan de détection incliné (angle θ). L'un des inserts est fixé au couvercle et l'autre insert est fixé la paroi de fond du bac, de telle sorte que les deux plans de détection des inserts sont face à face et délimitent entre eux un espace de faible largeur. Ce fantôme est utilisé notamment pour calculer la largeur de coupe. Ce fantôme présente au moins un inconvénient.. En effet, une fois le fantôme positionné par rapport à l'appareil d'imagerie, les inserts peuvent être utilisés pour calculer une largeur de coupe uniquement dans un plan. Pour calculer, une largeur de coupe dans un autre plan perpendiculaire, on est contraint de modifier la position du fantôme par rapport à l'appareil d'imagerie, ce qui complique et rend les opérations de contrôle fastidieuses à cause de la forme cylindrique du bac.

Objectifs de l'invention

Un objectif général de l'invention est de proposer une nouvelle solution technique pour le contrôle de qualité en imagerie 3D tomographique, qui simplifie et diminue considérablement la durée des opérations de contrôle de qualité, en termes notamment de manipulation par l'utilisateur et/ou de temps de calcul. Cette nouvelle solution est ainsi mieux adaptée aux besoins des utilisateurs d'appareil d'imagerie médicale, en leur facilitant le contrôle périodique des performances d'un appareil d'imagerie médicale.

Résumé de l'invention

L'invention a ainsi pour premier objet un fantôme qui est destiné à être utilisé pour le contrôle de qualité en imagerie tomographique, et qui est défini dans la revendication 1. Ce fantôme comporte un bac et au moins deux inserts fixés à l'intérieur du bac, chaque insert comportant un premier plan de calage, un plan de détection, qui est incliné par rapport audit premier plan de calage d'un angle ($\alpha$) inférieur à 90°, et au moins un deuxième plan de calage qui est opposé au sommet de l'angle ($\alpha$) et qui est perpendiculaire au premier plan de calage. Le bac est de forme parallélépipédique ; chaque insert est fixé à l'intérieur du bac d'une part de telle sorte que l'un des plans de calage de l'insert est parallèle à une première paroi du bac, et l'autre plan de calage de l'insert est parallèle à une autre paroi du bac perpendiculaire à ladite première paroi, et d'autre part de telle sorte que lesdits deuxièmes plans de calage des inserts sont perpendiculaires.

Plus particulièrement, mais de manière facultative selon l'invention, le fantôme de l'invention comporte les caractéristiques techniques additionnelles des revendications 2 à 18, prises isolément ou en combinaison.

L'invention a pour autre objet un procédé de préparation du fantôme précité. Selon ce procédé, on remplit le bac avec un liquide détectable dans le mode d'imagerie tomographique, c'est-à-dire un liquide permettant de former un contraste dans l'image. Pour l'utilisation du fantôme en imagerie TEP ou TDM, on utilise par exemple comme liquide détectable, une solution contenant un isotope présentant une activité connue de produit radioactif émetteur de positons. Pour l'utilisation du fantôme en imagerie TDM ou IRM, on utilise comme liquide détectable, une solution contenant un produit de contraste, et par exemple une solution iodée.

Plus particulièrement, lorsque le fantôme comprend un insert central, ce dernier est, de préférence, monté dans le bac après le remplissage dudit bac.

Plus préférentiellement encore, lorsque ledit insert central comporte deux cavités cylindriques fermées de diamètres différents, une fois le bac rempli, on remplit les deux cavités cylindriques avec du liquide contenu dans le bac, en aspirant l'air contenu dans ces cavités.

Plus particulièrement, lorsque le fantôme comporte un couvercle de fermeture du bac, une fois le bac rempli, et le cas échéant une fois les cavités cylindriques de l'insert remplies, on ferme le bac de manière étanche au moyen dudit couvercle.

L'invention a pour autre objet un procédé de contrôle de la qualité d'un appareil d'imagerie tomographique, et notamment d'un appareil d'imagerie médicale, au moyen d'un fantôme précité dont le bac contient un liquide détectable dans le mode d'imagerie tomographique. Selon le cas, le liquide détectable dans le mode d'imagerie tomographique peut être changé à chaque opération de contrôle qualité ou un fantôme contenant un liquide détectable dans le mode d'imagerie tomographique peut être utilisé pour réaliser plusieurs opérations de contrôle espacées dans le temps.

Selon ce procédé, on positionne le fantôme sur la table d'examen de l'appareil d'imagerie tomographique, en l'alignant avec les plans transverse (X,Z), sagittal (Y,Z) et coronal (X,Y) de l'appareil et de telle sorte que :

- la paroi de fond du bac est positionnée parallèlement au plan coronal horizontal (X,Y)
- la première paroi latérale verticale du bac est positionnée parallèlement au plan vertical transverse (X,Z)
- la troisième paroi latérale verticale est positionnée parallèlement au plan vertical sagittal (Y,Z).

Plus particulièrement, une fois le fantôme positionné sur la table d'examen et aligné avec les plans transverse, sagittal et coronal, on réalise les étapes successives suivantes :

(a) tomographie de l'intégralité du fantôme selon des plans de coupe transverse,
(b) de manière facultative, reconstruction par calcul du fantôme dans le plan sagittal et/ou dans le plan coronal, à partir des coupes transverses du fantôme acquises à l'étape (a),
(c) calcul d'un ou plusieurs paramètres de contrôle qualité à partir de coupes transverses du fantôme acquises à l'étape (a) et de manière facultative à partir de coupes sagittales et/ou coronales du fantômes reconstruites à l'étape (b).

Plus particulièrement, pour le calcul du ou des paramètres de contrôle qualité, on effectue préalablement une étape de recalage du volume du fantôme acquis par tomographie (Volume_CQ) sur un volume de référence (Volume_Ref), jusqu'à obtenir une transformation dans l'espace ($M_{ref}$) entre ces deux volumes, qui répond à un critère de similitude prédéfini.

L'invention a pour autre objet un ensemble comportant un fantôme visé précédemment et un programme de calcul d'un ou plusieurs paramètres de contrôle qualité ($P_i$) d'un appareil d'imagerie tomographique, ledit programme étant enregistré sur un support mémoire ou dans une mémoire, étant exécutable automatiquement par des moyens de calcul programmables, et permettant de calculer automatiquement un ou plusieurs paramètres de contrôle qualité, à partir au moins de coupes transverses qui ont été acquises par tomographie, au moyen dudit appareil d'imagerie tomographique, d'un fantôme.

Brève description des dessins

D'autres caractéristiques et avantages de l'invention apparaîtront plus clairement à la lecture de la description détaillée d'un exemple préféré de réalisation de l'invention, laquelle description détaillée est faite ci-après à titre d'exemple non limitatif et non exhaustif, et en référence aux dessins annexés sur lesquels :

- la figure 1 est une représentation en perspective d'un fantôme, conforme à une variante préférée de réalisation l'invention ;
- la figure 2 représente un insert d'angle du fantôme de la figure 1,
- la figure 3 est une vue côté de l'insert central du fantôme de la figure 1 ;
- la figure 4 est une vue de dessus de l'insert central du fantôme de la figure 1 ;
- la figure 5 est une représentation en perspective de l'insert central du fantôme de la figure 1 ;
- la figure 6 représente de manière schématique le_fantôme de la figure 1, positionné et orienté dans le repère (X, Y,Z) d'un appareil d'imagerie tomographique ;
- la figure 7 est un organigramme illustrant les principales étapes exécutées par un programme de calcul de l'invention ;
- la figure 8 est un schéma illustrant l'étape (72) de l'organigramme de la figure 7, et relatif à la reconstruction d'image dans le plan sagittal ;
- La figure 9 est un schéma illustrant l'étape (73) de l'organigramme de la figure 7, et relatif à l'étape de recalage automatique du volume du fantôme acquis par tomographie, sur un volume de référence ;
- La figure 10 représente trois exemples de coupes transverse (a), sagittale (b) et coronale (c), utilisées pour le calcul des paramètres suivants : rapports Signal/Bruit, Uniformité, facteurs d'agrandissement en X, Y et Z ;
- La figure 11 représente un exemple de coupe transverse utilisée pour le calcul des résolutions spatiales en X et en Y ;
- La figure 12 représente des exemples de profils de niveaux de gris résultant respectivement des différentes étapes successives de calcul de la résolution spatiale ;
- La figure 13 est un exemple de plan de coupe transverse, utilisé pour le calcul de la largeur de coupe ;
- La figure 14 représente un exemple de profil de niveaux de gris utilisé pour le calcul de la largeur de coupe ;
- La figure 15 représente deux exemples de coupes sagittales reconstruites par MPR et utilisées pour le calcul des contrastes des volumes chauds et froids ;
- La figure 16 représente des exemples de coupes transverses utilisées pour le calcul de la précision relative de comptage,
- La figure 17 est une représentation en perspective d'un fantôme, conforme à une autre variante de réalisation de l'invention.

Description détaillée

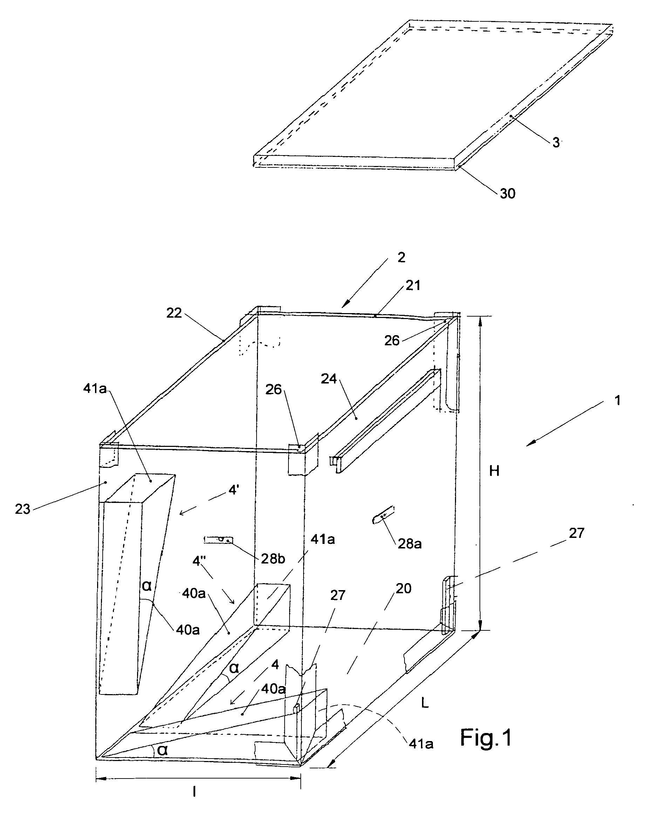

On a représenté sur les figures 1 à 6, une variante préférée de réalisation d'un fantôme 1, qui a été plus spécialement développé pour le contrôle de qualité en imagerie TEP, mais qui peut également être utilisé dans d'autres types d'imagerie par tomographie (IRM, TEMP, TDM ...). Par souci de simplification de l'exposé, la suite de la description est axée dans le domaine de l'imagerie TEP ; l'invention n'est toutefois pas limitée à ce seul domaine particulier d'imagerie tomographique, l'homme du métier pouvant transposer l'enseignement ci-après dans les autres types d'imagerie tomographique. Le fantôme de l'invention est principalement utilisé pour le contrôle qualité d'un appareil d'imagerie tomographique médicale, mais peut également être utilisé en dehors du domaine médical, pour le contrôle de qualité de tout type d'appareil imagerie par tomographie.

Structure du fantôme

Le fantôme 1 comporte un bac 2 et un couvercle 3 amovible adaptable sur le bac 2 et permettant une fermeture du bac 2. De préférence, la couvercle 3 (ou le bac 2) est pourvu sur toute sa périphérie d'un joint d'étanchéité 30, de telle sorte qu'une fois le couvercle 3 positionné et fixé sur le bac 2, il en assure une fermeture étanche. Ce bac est destiné à contenir un liquide permettant de former un contraste dans l'image. Pour une utilisation en imagerie TEP (ou en imagerie TEMP), ce bac 2 est par exemple destiné à contenir une solution contenant un isotope tel que par exemple l'isotope $^{18}F$ couramment utilisé en TEP pour marquer les produits pharmaceutiques utilisés pour le diagnostic du cancer. On peut également utiliser une solution contenant tout autre isotope radioactif émetteur de positons, par exemple à durée de vie plus longue, ce qui permet d'utiliser ce type de solution pour réaliser plusieurs opérations de contrôle qualité espacées dans le temps, sans être obligé de changer le liquide contenu dans le bac du fantôme. Pour une utilisation du fantôme 1 en imagerie TDM ou IRM, on remplacera cette solution radioactive par une solution adaptée à ces types d'imagerie, et par exemple une solution contenant un produit de contraste, et plus particulièrement une solution iodée.

Le bac 2 forme un parallélépipède rectangle de hauteur (H), de longueur (L) et de largeur (I), et comprend une paroi de fond 20, et quatre paroi latérales 21 à 24. Le bac 2 et son couvercle 3 peuvent être réalisés dans tout type de matériau compatible avec un appareil d'imagerie TEP, et sont par exemple en acrylique ou en PVC.

Le fantôme 1 comporte trois inserts 4, 4', 4" qui permettent de calculer des paramètres de qualité, qui seront détaillés ultérieurement.

Chaque insert 4, 4', 4" est réalisé dans un matériau compatible avec un appareil d'imagerie TEP, et est par exemple, mais non nécessairement selon l'invention, en PVC ou acrylique (notamment PMMA).

Dans un souci de simplification de la fabrication du fantôme, ces trois inserts 4, 4', 4" sont de préférence identiques, mais pourraient, dans une autre variante de réalisation, être différents.

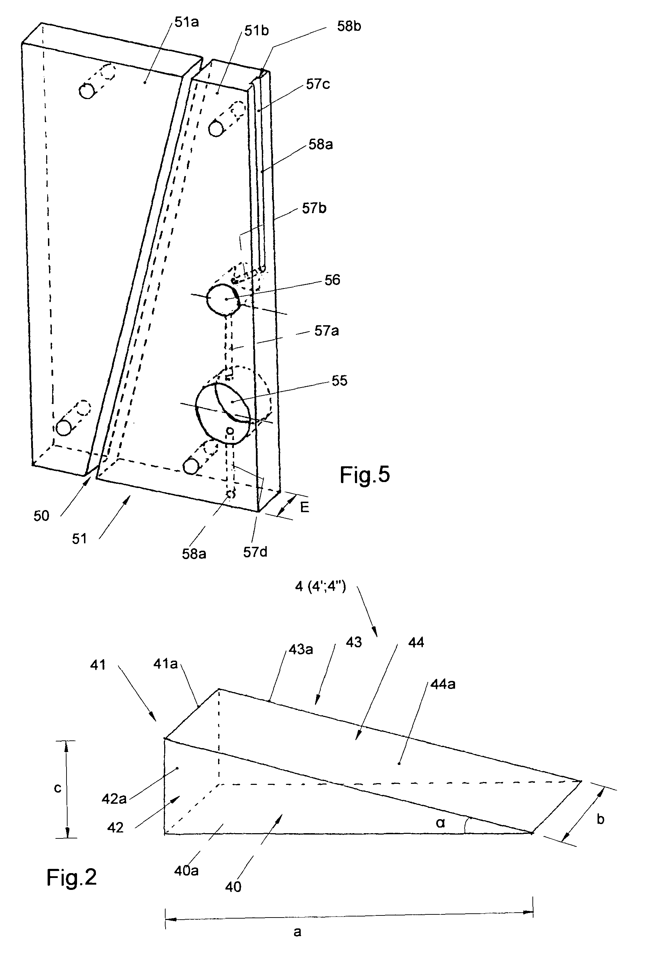

Dans l'exemple préféré illustré sur la figure 2, chaque insert 4, 4' 4" est constitué par une pièce pleine présentant, en section transversale, une forme de triangle rectangle, et comprend :

- une première face 40 formant un premier plan de calage principal 40a,
- trois autres face 41 à 43 formant chacune un plan de calage secondaire 41a à 43a, qui est perpendiculaire au plan de calage principal 40a,
- une face de détection 44 formant un plan de détection 44a qui est incliné d'un angle aigu $\alpha$ (angle non nul inférieur à 90°) par rapport au plan de calage principal 40a.

L'invention n'est toutefois pas limitée à cette variante préférée de section transversale en forme de triangle rectangle, mais peut notamment être mise en oeuvre avec tout insert comportant un plan de calage principal 40a, au moins un plan de détection 41a incliné, par rapport au plan de calage principal 40a d'un angle aigu $\alpha$ (angle $\alpha$ non nul inférieur à 90°) et au moins un plan de calage secondaire 41a, qui est opposé au sommet de l'angle ($\alpha$) et qui est perpendiculaire au plan de calage principal 40a.

Les inserts 4, 4', 4" sont fixés, par tout moyen de fixation approprié, et par exemple par collage, à l'intérieur du bac 2. Dans l'exemple particulier et non limitatif de la figure 1, les inserts 4, 4', 4"sont fixés dans des angles différents du bac 2, de telle sorte que le plan de calage principal 40a de chaque insert est positionné contre une des parois du bac, et qu'au moins un des plans de calage secondaire (41a, 42a, 43a) de l'insert est positionné contre une autre paroi adjacente du bac. En outre, les plans de calage secondaires 41a des inserts, c'est-à-dire les plans de calage opposés au sommet de l'angle ($\alpha$) sont perpendiculaires entre eux.

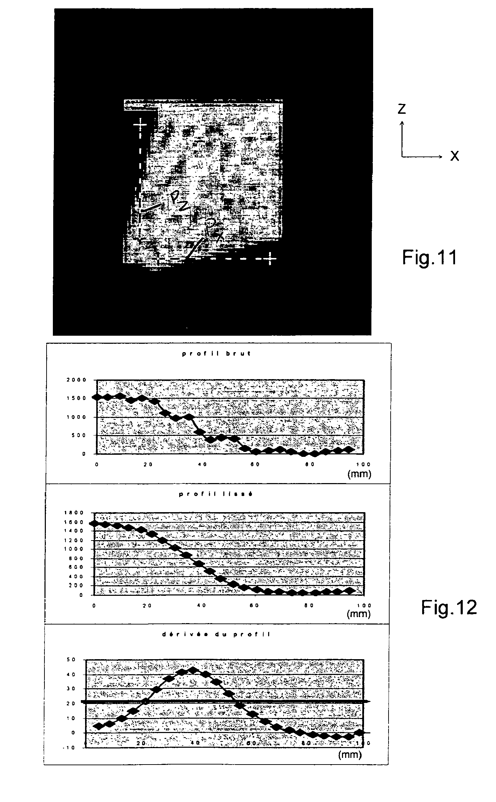

Plus particulièrement, dans l'exemple de la figure 1, l'insert 4' et l'insert 4" inserts sont positionnés l'un par rapport à l'autre de telle sorte qu'au moins un plan de coupe perpendiculaire aux plans de calage 41a des deux inserts (c'est-à-dire dans cet exemple un plan de coupe parallèle à la paroi 22 du bac) est sécant avec les plans de détection 44a des deux inserts 4', 4". Cette caractéristique permet avantageusement, lors d'une acquisition tomographique, d'avoir dans une seule et même coupe image le profil des deux plans de détection 44a des deux inserts 4' et 4", tel que cela est illustré sur la coupe transverse de la figure 11.

En particulier, dans l'exemple illustré sur la figure 1, l'insert 4 est fixé dans le fond du bac 2, de telle sorte que son plan de calage principal 40a est posé contre la paroi de fond 20 du bac, et que ces deux plans de calage secondaires 41a et 43a sont positionnés respectivement contre la paroi latérale verticale 24 et la paroi verticale 23 du bac 2. L'insert 4' est fixé contre les deux parois latérales 22 et 23 du bac 2, de telle sorte que son plan de calage principale 40a est positionné contre la paroi latérale verticale 23 du bac, et que son plan de calage secondaire 43a est positionné contre la paroi latérale verticale 22. L'insert 4" est fixé dans le fond du bac 2, de telle sorte que son plan de calage principal 40a est posé contre la paroi de fond 20 du bac, et que ces deux plans de calage secondaires 41a et 42a sont positionnés respectivement contre la paroi latérale verticale 21 et la paroi verticale 22 du bac 2.

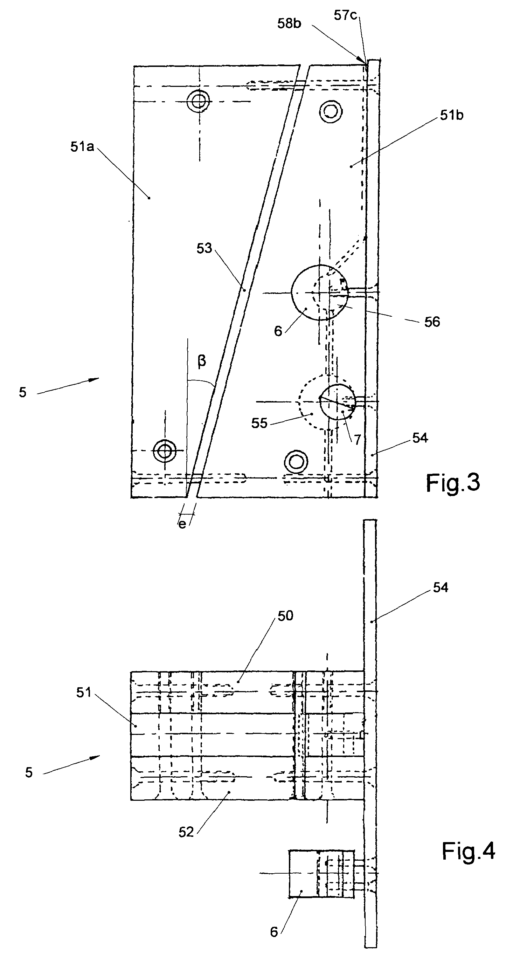

Dans le but de pouvoir calculer d'autres paramètres de qualité qui seront détaillés ultérieurement, le fantôme 2 est de préférence équipé d'un insert central supplémentaire 5 amovible, qui est représenté sur les figures 3 à 6, et qui va à présent être détaillé.

Cet insert 5 comporte trois plaques 50, 51 et 52 assemblées entre elles, en sorte de former un ensemble parallélépipédique, la plaque 51 centrale étant prise en sandwich entre les deux plaques externes 50 et 52. Ces plaques 50, 51 et 52 sont réalisées dans un matériau compatible avec le mode imagerie tomographique, tel que par exemple de l'acrylique (notamment PMMA) ou du PVC. L'ensemble formé par les trois plaques 50, 51 et 52 est en outre fixé perpendiculairement sur une plaque de base 54 de faible épaisseur.

Chaque plaque 50, 51, 52 comporte deux parties distinctes, qui sont assemblées, en étant séparées sur toute leur hauteur, par une fente, inclinée et de largeur constante prédéfinie (e). Une fois les trois plaques assemblées entre-elles, elles forment un bloc parallélépipédique comportant une fente 53 traversante, inclinée et de largeur constante prédéfinie (e). Cette fente 53 débouche dans les deux faces principales dudit bloc parallélépipédique qui correspondent au deux faces externes des deux plaques externes 50 et 51, et dans les deux faces supérieure et inférieure dudit bloc. Ainsi, lorsque l'insert 5 est plongé dan un liquide contenu dans le bas, cette fente 53 est remplie dudit liquide.

De manière additionnelle et facultative, l'insert 5 comporte également deux cavités cylindriques fermées 55 et 56, parallèles et de diamètres différents. Chaque cavité cylindrique 55, 56 est constituée par un trou cylindrique traversant réalisé dans la plaque centrale 51, et fermé à ses deux extrémités respectivement par les deux plaques externes 50 et 52.

Les deux cavités 55 et 56 communiquent entre elles par un canal 57a de faible diamètre percé dans l'épaisseur de la plaque centrale 51. La cavité cylindrique supérieure 56 communique en outre avec un canal 57b de faible diamètre qui est percé dans l'épaisseur de la plaque centrale 51, et qui débouche, à son extrémité opposée à la cavité 56, dans une rainure 57c réalisé dans un bord longitudinal de la plaque 51. Cette rainure 57c se prolonge et débouche dans le bord supérieur de la plaque 51 et délimite ainsi, avec la plaque de base 54, un conduit présentant, à son extrémité supérieure, une ouverture d'évacuation 58b.

La cavité cylindrique inférieure 55 communique avec un canal 57d, de faible diamètre, qui est percé dans l'épaisseur de la plaque 51, et qui débouche dans le bord inférieur de cette plaque 51 (orifice d'admission 58a) Ces canaux 57a, 57b ; 57c et 57d permettent de remplir par aspiration les deux cavités cylindriques 55 et 56 avec un liquide, contenu dans le bac 2, en utilisant par exemple une seringue ou équivalent. En aspirant l'air contenu dans les chambres 55 et 56, au moyen de cette seringue (ou de tout autre moyen d'aspiration équivalent), par l'intermédiaire de l'orifice d'évacuation supérieur 57b, on remplit facilement et rapidement par aspiration les deux cavités 55 et 56 avec du liquide contenu dans le bac 2 et pénétrant par l'orifice d'admission inférieur 57a. Ces cavités serviront de volumes dits "chauds".

Enfin, de manière optionnelle et facultative, le fantôme 2 est de préférence également équipé de deux inserts cylindriques pleins 6 et 7, qui sont fixés l'un au dessus de l'autre à la plaque de base 54, en sorte d'avoir la même orientation que les cavités cylindriques 55 et 56. L'insert 6 supérieur a le même volume que la cavité cylindrique inférieure 55 et l'insert 7 inférieur a le même volume que la cavité cylindrique supérieure 56. Ces inserts cylindriques serviront de volumes dits "froids".

La plaque de base 54 supportant les inserts 5, 6 et 7 précités est apte à être insérée verticalement et de manière amovible dans deux rainures verticales 26 (figure 1) réalisées dans les parois latérales opposées 21 et 24 du bac 2 du fantôme 1. Le fantôme 1 comporte en outre deux cales 27, fixées dans le fond du bac 2 et dans les angles de la paroi 24 avec les deux parois 21 et 23, et permettant de surélever la plaque de base 54 par rapport au fond 20 du bac 2, et de positionner les inserts 5, 6 et 7, portés par cette plaque 54, au dessus des inserts 4 et 4" fixés dans le fond 20 du bac 2.

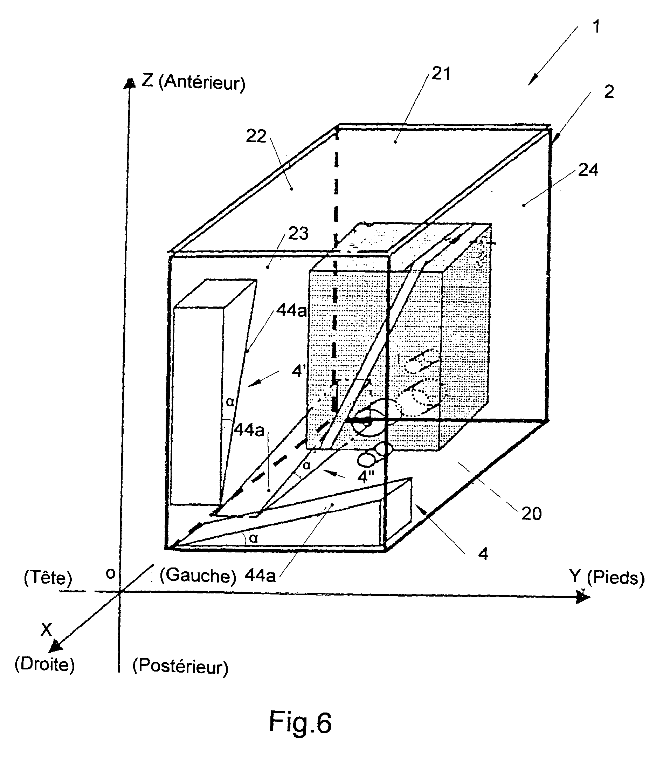

Lorsque la plaque de base 54 est insérée dan les deux rainures 26 du bac 2, et est amenée en butée contre les deux cales 27, les inserts 5, 6 et 7 sont positionnés à l'intérieur du bac 2, au dessus du niveau des inserts 4 et 4", de telle sorte que (figure 6) :

- la fente 53 de l'insert 5 est orientée perpendiculairement aux plans des parois latérales verticales 21 et 23, et est inclinée d'un angle β par rapport à un axe vertical perpendiculaire à la paroi de fond 20 du bac 2,
- les deux cavités cylindriques 55 et 56 de l'insert 5 et les deux inserts cylindriques pleins 6 et 7 correspondant sont orientés perpendiculairement aux plans des parois latérales verticales 21 et 23.

De préférence, mais facultativement selon l'invention, le fantôme 1 comporte des moyens de contrôle de l'horizontalité de la paroi de fond 20 du bac 2. Ces moyens comportent par exemple deux niveaux à bulle 28a, 28b fixés, l'un 28a sur la paroi verticale 24 du fantôme, et l'autre 28b sur la paroi verticale 23 du fantôme.

Dans un exemple précis de réalisation, donné à titre d'exemple non limitatif de l'invention, les principales dimensions optimales du fantôme étaient les suivantes.

La hauteur H̱ du bac 2 valait 230 mm ; la longueur Ḻ du bac 2 valait 150mm et était suffisamment faible pour permettre d'acquérir l'intégralité du bac 2, en une seule série de coupes natives. La largeur I̱ du bac 2 valait 190mm. Les dimensions a, b et c de chaque insert 4, 4', 4" (figure 2) valaient respectivement 150 mm, 30mm et environ 37mm. L'angle α des inserts 4, 4', 4" et l'angle β de la fente 53 de l'insert 5 étaient de 14°. Plus généralement, ces angles α et β peuvent être compris entre 0° et 90°. Néanmoins, mais de manière non limitative de l'invention, on choisira de préférence des valeurs d'angles α et β les plus faibles possible et compatibles avec la mécanique du fantôme et les possibilités de calcul. Ainsi, les valeurs des angles $\alpha$ et $\beta$ sont de préférence inférieures à 45°. La largeur e de la fente 53 valait par exemple 3,8 mm pour un appareil d'imagerie TEP présentant une largeur de coupe de l'ordre de 4mm. L'insert 5 formait un bloc parallélépipédique de 188mmx60mmx102mm. Le diamètre de la cavité 55 et de l'insert 6 valait 25mm ; le diamètre de la cavité 56 et de l'insert 7 valait 10mm. L'épaisseur E (figure 5) de la plaque centrale 51, correspondant à la hauteur des cavités cylindrique 55, 56, valait environ 20mm.

Préparation du fantôme

Le fantôme 1 est préparé et utilisé de la manière suivante.

Le couvercle 3 et la plaque de base 54 portant les inserts 5, 6 et 7 sont retirés du bac 2, les autres inserts 4, 4' 4", étant fixés à l'intérieur du bac 2.

On remplit le bac 2 du fantôme 1 avec un volume d'eau pure (par exemple avec 4250 ml dans le cas du dimensionnement particulier précité) On ajoute et on mélange au volume d'eau pure un faible volume (par exemple environ 1ml) d'une solution contenant un isotope du type $^{18}$F, et présentant une activité connue (par exemple 13 kBq/ml). Cette solution est par exemple un dérivé du glucose, tel que du FDG (Fluor-DéoxyGlucose), marqué au $^{18}$F. Ensuite, on remet en place la plaque de base 54 portant les inserts 5, 6 et 7, en la glissant dans les rainures 26 du bac 2, jusqu'à ce qu'elle soit en butée contre les cales 27. Les inserts 5, 6 et 7 sont complètement immergés dans le liquide contenu dans le bac, la fente traversante 53 de l'insert 5 étant remplie de ce liquide.

Ensuite, on remplit les cavités cylindriques 55 et 56 de l'insert 5, avec du liquide contenu dans le bac 2 et pénétrant dans l'insert 5 par l'ouverture d'admission inférieure 58a, en aspirant, par l'ouverture d'évacuation supérieur 58b, et au moyen d'une seringue et d'un cathéter ou équivalent, l'air contenu dans les canaux 57a, 57b, 57c, 57d et dans les deux cavités cylindriques 55 et 56.

Ensuite le bac 2 est refermé de manière étanche avec le couvercle 3, et le fantôme 2 est prêt à être utilisé de la manière décrite ci-après.

Après utilisation du fantôme 1, celui-ci est vidangé très simplement en retirant le couvercle 3 et en déversant le liquide contenu dans le bac 2 (y compris celui contenu dans les cavités cylindriques 55 et 56).

Comparativement à des solutions connues de l'art antérieur, dans lesquelles il faut par exemple remplir avec une solution radioactive (puis vidanger) des tubes longs et de très faible diamètre devant être insérés axialement dans un fantôme, généralement de forme cylindrique, les opérations de remplissage et de vidange précitées du fantôme 1 de l'invention sont avantageusement plus rapides et plus simples.

Positionnement du fantôme sur la table d'examen

Une fois le fantôme 1 rempli, on le positionne sur la table d'examen de l'appareil d'imagerie TEP, dont on souhaite contrôler la qualité d'image. Il convient de noter que dans une autre variante de l'invention, un fantôme rempli avec un liquide détectable peut être utilisé pour réaliser plusieurs opérations de contrôle ; dans ce cas, on utilise un fantôme qui est déjà rempli, et dont le couvercle est de préférence scellé.

Sur la figure 6, on a symbolisé par le repère tridimensionnel (X,Y,Z) le repère réel de l'appareil d'imagerie TEP. Le plan vertical (X,Z) correspond au plan des coupes transverses (encore désignées coupes natives) de l'appareil d'imagerie TEP.

Pour réaliser le contrôle de qualité d'image d'un appareil d'imagerie TEP, on positionne le fantôme 1 sur la table d'examen de l'appareil d'imagerie TEP en alignant les parois du bac 2 du fantôme 1 avec le repère orthogonal (X, Y,Z) de l'appareil d'imagerie, tel que cela est illustré sur la figure 6. Cet alignement est effectué en utilisant les lasers d'alignement transverse, sagittal et coronal qui, de manière usuelle, équipent ce type d'appareil d'imagerie.

En particulier, lorsque ces opérations d'alignement sont achevées :

- la paroi de fond 20 est positionnée parallèlement au plan horizontal (X,Y) [plan parallèle aux coupes coronales] ;
- la paroi latérale verticale 22 est positionnée parallèlement au plan vertical (X,Z)) [plan parallèle aux coupes natives] ;
- les deux parois latérales verticales opposées 21 et 23 sont positionnées parallèlement au plan vertical (Y,Z) [plan parallèle aux coupes sagittales] .

Lorsque le fantôme 1 comporte des niveaux à bulle, on vérifie également l'horizontalité du bac 2 et on rectifie le cas échéant l'inclinaison de ce bac 2, de telle sorte que sa paroi de fond 20 soit horizontale.

Ces opérations de positionnement et d'alignement du bac 2 du fantôme dans le repère (X,Y,Z) de l'appareil d'imagerie TEP sont avantageusement simples et rapides à effectuer, et permettent en outre d'obtenir simultanément les orientations appropriées ci-après de chaque insert 4, 4', 4", 5, 6 et 7 . En effet, lorsque le bac 2 est parfaitement aligné avec le repère (X,Y, Z) de l'appareil d'imagerie :

- le plan de détection 44a de l'insert 4 fait un angle aigu α avec le plan coronal (X,Y) et est orienté perpendiculairement au plan sagittal (Y,Z) ;
- le plan de détection 44a de l'insert 4' fait un angle aigu α avec le plan sagittal (Y,Z) et est orienté perpendiculairement au plan transverse (X,Z) ;
- le plan de détection 44a de l'insert 4" fait un angle aigu α avec le plan coronal (X,Y) et est orienté perpendiculairement au plan transverse (X,Z).
- la fente 53 de l'insert forme un angle β avec le plan transverse (X,Z),
- les axes longitudinaux des cavités cylindriques 55 et 56 de l'insert 5, et les axes longitudinaux des inserts cylindriques 6 et 7 sont parallèles au plan transverse (X,Z) et perpendiculaires au plan sagittal (Y,Z).

Acquisition et traitement des données pour le contrôle de qualité

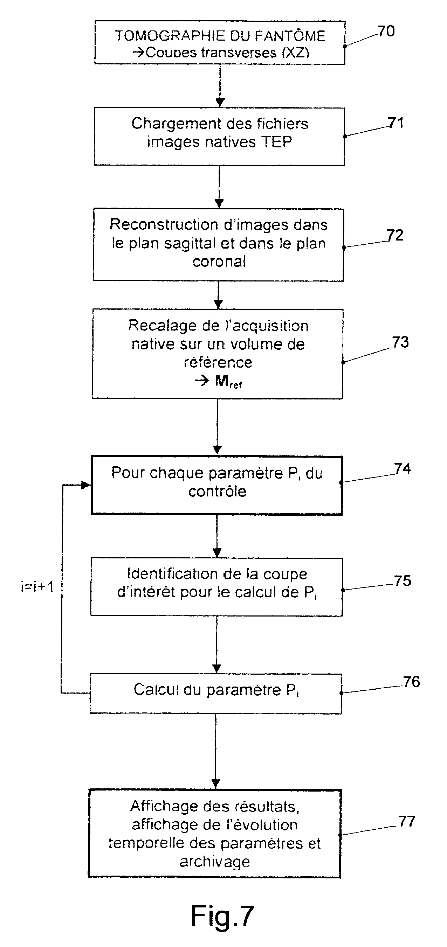

Une fois que le fantôme 1 a été préparé et positionné sur la table d'examen de l'appareil d'imagerie TEP, et aligné avec le repère tridimensionnel orthogonal (X,Y,Z) de l'appareil d'imagerie TEP, les opérations d'acquisition et de traitement de données pour le calcul automatique de plusieurs paramètres de contrôle qualité, qui seront détaillés ultérieurement, peuvent commencer.
Les principales étapes de ces opérations d'acquisition et de traitement de données vont à présent être détaillées en référence à l'organigramme de la figure 7.

Etape 70 :

De manière connue en soi, l'opérateur réalise, au moyen de l'appareil d'imagerie TEP, une tomographie de l'intégralité du fantôme 1, selon des plans de coupe transverse [plan parallèle au plan (X,Z)] d'épaisseur prédéfinie et caractéristique de l'appareil d'imagerie. Toutes les images natives (coupes transverses) correspondant à ces plans de coupe transverse sont sauvegardées dans des fichiers de l'appareil d'imagerie.

Etapes 71 à 77

Les étapes suivantes 71 à 77 sont réalisées par un programme de traitement de données qui est spécifique de l'invention. Ce programme est sauvegardé par exemple dans une mémoire d'un calculateur programmable, qui est apte à exécuter ledit programme, pour réaliser automatiquement les étapes 71 à 77 de la figure 7 qui vont à présent être détaillées.

Etape 71

Les fichiers contenant les images natives (coupes transverses du fantôme) résultant de l'étape d'acquisition 70 sont chargés en mémoire du calculateur.
Il convient de noter que cette étape 71 et les étapes suivantes peuvent être réalisées immédiatement à la suite de l'étape 70 d'acquisition initiale ou peuvent être différées dans le temps.
Egalement, pour les opérations de chargement en mémoire des fichiers, le calculateur programmable utilisé pour réaliser ces étapes 71 et suivantes peut être connecté localement à l'appareil d'imagerie médicale ou au contraire être utilisé sur un site plus ou moins distant. Lorsque le calculateur utilisé est distant de l'appareil d'imagerie, les fichiers contenant les images natives peuvent être téléchargés via un réseau de communication, et notamment via le réseau mondial Internet ; également on peut utiliser un support mémoire amovible, sur lequel sont stockés temporairement les fichiers contenant les images natives, et qui est transporté depuis le site de l'appareil d'imagerie jusqu'au site du calculateur, en vu d'un chargement en local de ces fichiers en mémoire du calculateur.

Etape 72

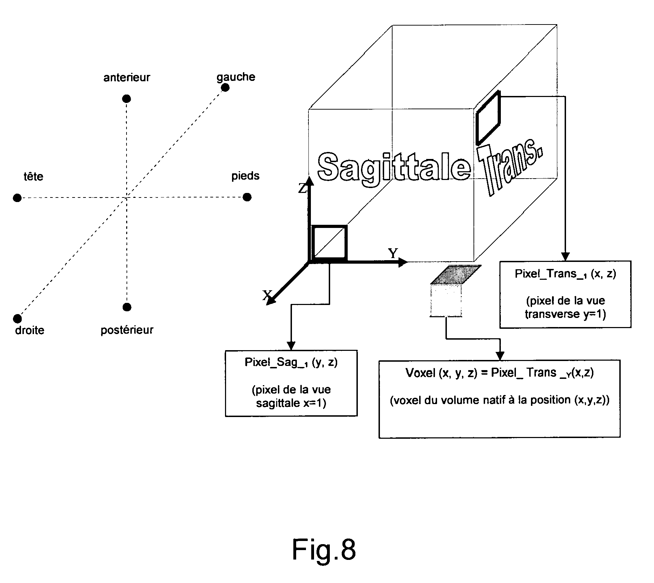

A partir des fichiers contenant les images natives, le calculateur programmable reconstruit automatiquement les images du fantômes dans le plan sagittal (Y,Z) et dans le plan coronal (X,Y). Pour cette reconstruction, l'homme du métier met en oeuvre un simple algorithme de construction multiplanaire connu en soi dans le domaine de l'imagerie par tomographie sous le nom de MPR.
Dans une autre variante plus simple la reconstruction peut être effectuée uniquement dans le plan sagittal ou uniquement dans le plan coronal, lorsque les paramètres calculés ultérieurement n'utilisent pas les coupes respectivement coronales ou sagittales.
Un exemple d'algorithme de reconstruction en pseudo-code est donné ci-après, en lien avec la figure 8, pour une reconstruction dans le plan sagittal.

Algorithme reconstruction

```
Pour(x=taille_x → x=1)
     Pour(y=1 → y=taille_y)
          Pour(z=1 → z=taille_z)
          Pixel_Sag_x(y,z)=Voxel(x,y,z)
          Fin pour
     Fin pour
 Fin pour
```

Etape 73

Cette étape a pour but de calculer automatiquement la position et l'orientation réelles du fantôme 1 dans le repère (OX,OY,OZ) des coupes natives en vue de détecter les éventuels écarts de position et d'orientation du fantôme 1 dans ce repère (OX,OY,OZ) et en particulier les éventuels écarts d'alignements du fantôme 1 avec les axes X, Y et Z. Cette étape est préférentielle, mais facultative. Lorsqu'elle est mise en oeuvre, elle permet notamment, lors du calcul ultérieur de certains paramètres de qualité, de tenir compte et de corriger les éventuels erreurs ou manques de précision dans d'alignement du fantôme sur la table d'examen. Cela permet avantageusement de rendre les opérations d'alignement du fantôme moins contraignantes.

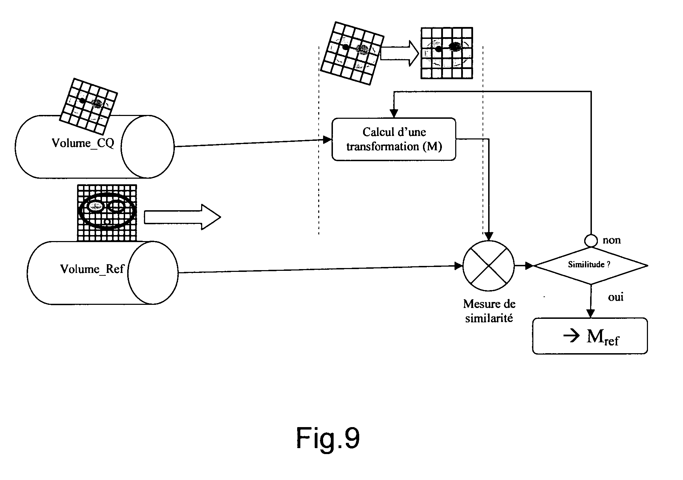

En référence à l'étape 7 et à la figure 9, le programme de calcul compare le volume du fantôme 1 (référencé Volume_CQ sur la figure 9) qui a été acquis par tomographie avec un volume de référence préenregistré (référencé Volume_Ref sur la figure 9). Ce volume de référence peut être un modèle numérique du fantôme ou une tomographie de référence du fantôme 1.

Plus particulièrement, le programme effectue automatiquement par itérations successives un recalage du volume acquis par tomographie (Volume_CQ) sur le volume de référence (Volume_Ref), en sorte d'obtenir une transformation dans l'espace $M_{ref}$ entre ces deux volumes, qui répond à un critère de similitude prédéfini. Par exemple, ce critère de similitude est l'information mutuelle qui est maximisée grâce à une optimisation type Powell.

Pour de plus amples explications sur l'information mutuelle appliquée à l'imagerie médicale, l'homme du métier peut se référer à la publication suivante :

IWells III W.M. Viola P. Atsumi H. Nakajima S. Kikinis R. Multimodal Volume Registration by Maximisation of Mutual Information. Medical Image Analysis 1, 35-51. 1997.

Pour de plus amples explications sur l'optimisation Powell, l'homme du métier peut se référer à la publication suivante :

Press W.H. Flannery B. P. Teukolski S. A. Vetterling W. R. Numerical Recipies in C. Cambridge University Press. 1988

L'information mutuelle mesure la dépendance statistique des deux variables aléatoires constituées par les deux volumes (Volume_CQ et Volume_Ref). L'information mutuelle est nulle si les variables sont indépendantes, et augmente si la dépendance augmente.

L'algorithme d'optimisation Powell permet d'obtenir les trois angles de rotation et la translation dans l'espace de la TEP en vue de déplacer le volume du fantôme acquis par tomographie (Volume_CQ) pour une information mutuelle avec le volume de référence (Volume-Ref), ce qui correspond à l'alignement des volumes. Ces quatre paramètres permettent ensuite d'obtenir la zone d'intérêt adéquate dans le fantôme acquis par tomographie (Volume_CQ) pour le calcul de chaque paramètre $P_i$, car ces zones d'intérêts sont clairement identifiées dans le volume de référence (Volume_Ref).

La transformation $M_{ref}$ est de la forme :

$$P_{CQ} = M_{REF \rightarrow CQ} \cdot P_{REF}$$

avec

$$P_{CQ} = \begin{bmatrix} x_{CQ} \\ y_{CQ} \\ z_{CQ} \\ 1 \end{bmatrix} \quad , \quad P_{REF} = \begin{bmatrix} x_{REF} \\ y_{REF} \\ z_{REF} \\ 1 \end{bmatrix} \quad \text{et} \quad M_{REF \to CQ} = \left( M_{CQ \to REF} \right)^t = \begin{pmatrix} r_{11} & r_{12} & r_{13} & t_1 \\ r_{21} & r_{22} & r_{23} & t_2 \\ r_{31} & r_{32} & r_{33} & t_3 \\ 0 & 0 & 0 & 1 \end{pmatrix}$$

où $P_{CQ}$ est un point du volume acquis (Volume_CQ), $P_{REF}$ est un point du de référence (Volume_Ref), $r_{ab}$ sont les éléments relatifs à la rotation selon les axes (XD,Y,Z) du repère de l'appareil d'imagerie, et $t_a$ sont les éléments de la translation.

Il convient de souligner que ce calcul de la transformation $M_{ref}$, par recalage sur un volume de référence du fantôme, est avantageusement simplifié par le fait que dans le cadre de l'invention le fantôme 1 comporte un bac 2, qui forme un parallélépipède, et de préférence un parallélépipède rectangle, comparativement par exemple à la mise en oeuvre d'un fantôme du type cylindre, présentant une symétrie de révolution selon au moins un axe, ce qui rend plus délicat le recalage.

Etapes 74 à 76

Ces étapes 74 à 76 sont mises en oeuvre pour le calcul de chaque paramètre de contrôle qualité $P_i$.
L'étape 75 d'identification de la coupe d'intérêt pour le paramètre $P_i$ concerné, dépend d'une part de ce paramètre (tel que cela apparaîtra plus clairement à la lumière des exemples données ci-après de paramètre de contrôle qualité), et d'autre part est réalisée en prenant en compte la position et l'orientation du fantôme, après recalage (c'est-à-dire en prenant en compte la transformation $M_{ref}$ calculée à l'étape précédente).
Selon le type de paramètre, lors de l'étape de calcul 76, on intègre dans le calcul une correction d'angle, par exemple pour corriger la valeur des angles $\alpha$ (pour les inserts 4, 4', 4") ou $\beta$ (pour la fente 53 de insert 5).

Etape 77

Les valeurs des paramètres calculées sont affichées sur un écran, de préférence avec un historique de l'évolution dans le temps de chaque paramètre, et sont de préférence archivées en mémoire.
Grâce à la simplicité et la rapidité de mise en oeuvre du fantôme 1 de l'invention, ces opérations de contrôle qualité de l'appareil d'imagerie TEP peuvent être effectuées rapidement et avec une fréquence importante (par exemple une fois par jour, une fois par semaine, ...) par l'utilisateur de l'appareil d'imagerie. En outre l'invention permet, en équipant chaque centre médical utilisateur d'un appareil d'imagerie TEP avec un fantôme 1 de l'invention et le programme de calcul de paramètres de qualité associé, de réaliser des études multicentriques en suivant et en comparant les évolutions dans le temps de la qualité des différents appareils d'imagerie utilisés dans les différents centres médicaux

Exemples de paramètres de contrôle qualité

Différents paramètres de contrôle qualité vont à présent être détaillés, étant précisé que la liste de ces paramètres n'est pas exhaustive, et que d'autres paramètres de qualité, calculés à partir de coupes du fantômes 1 de l'invention, peuvent être définis par l'homme du métier, sans pour autant sortir du cadre de l'invention.

Rapport Signal/Bruit (RSB)

Trois paramètres RSB peuvent être calculés : un paramètre $RSB_{transverse}$ sur une coupe native transverse [parallèle au plan (X,Z)], et deux paramètres $RSB_{sagittal}$, $RSB_{coronal}$ respectivement sur des plans de coupe sagittale [parallèle au plan (Y,Z)] et coronale [parallèle au plan (X,Y)] reconstruites lors de l'étape 72 précitée.
Chaque paramètre RSB est mesuré et calculé dans une région de la coupe, dit région d'intérêt, dont la position est telle que ladite région d'intérêt est libre, c'est-à-dire dans une région de la coupe dans laquelle le fantôme contient uniquement du liquide.
En pratique, pour le paramètre $RSB_{transverse}$ cette région d'intérêt correspond à une région centrale d'un plan de coupe transverse située entre la paroi 22 du fantôme 1 et l'insert central 5. Pour le paramètre $RSB_{sagittal}$, cette région d'intérêt correspond à une région centrale d'un plan de coupe sagittale située entre la paroi 23 du fantôme et l'insert 5. Pour le paramètre $RSB_{coronal}$, cette région d'intérêt correspond à une région centrale d'un plan de coupe coronale située entre la paroi de fond 20 du fantôme et l'insert 5.

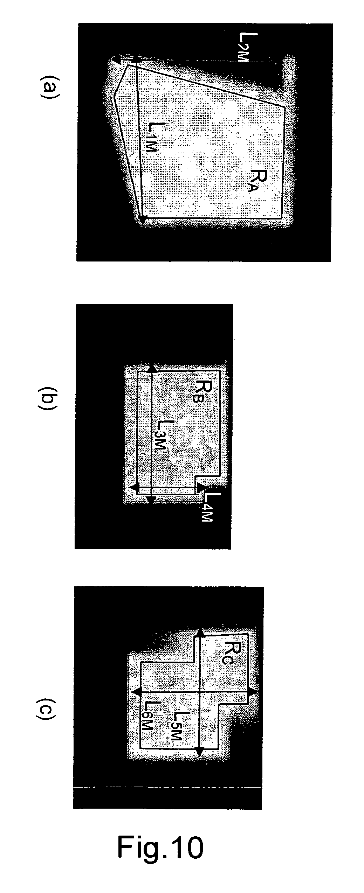

On a représenté sur la figure 10, trois exemples de plan de coupe transverse, sagittale et coronale pouvant être utilisés pour calculer respectivement les paramètres $RSB_{transverse}$, $RSB_{sagittal}$, $RSB_{coronal}$, la région d'intérêt pour le calcul apparaissant en clair, et les régions noires correspondant selon le cas aux inserts 4, 4' ou 4". Dans chaque image, la région d'intérêt est déterminée par seuillage.

Pour chaque plan de coupe transverse, sagittale, coronale, le paramètre RSB est calculé par exemple au moyen de la formule (1) suivante :

$$(1) \quad RSB = \frac{\overline{GL}}{SD}$$

dans laquelle $\overline{GL}$ est la moyenne des niveaux de gris calculée dans la région d'intérêt et SD est la déviation standard des niveaux de gris calculée dans la région d'intérêt.

Uniformité (U)

Trois paramètres U d'uniformité peuvent être calculés au moyen des mêmes plans de coupe transverse, sagittale et coronale, que ceux précédemment décrits pour les paramètres RSB, et au moyen de la formule (2) suivante :

$$(2) \quad U = \frac{1}{2} \frac{GL_{max} + GL_{min}}{GL_{max} - GL_{min}}$$

dans laquelle $GL_{max}$ et $GL_{min}$ représentent respectivement le maximum et le minium de niveau de gris dans la région d'intérêt, après filtrage de ladite région au moyen d'un filtre passe-bas, permettant de minimiser les fluctuations dans l'image dues au bruit.

Facteurs d'agrandissement en X, Y et Z

Trois facteurs d'agrandissement selon les 3 axes X, Y et Z sont calculés au moyen des mêmes plans de coupe transverse, sagittale et coronale, que ceux précédemment décrits pour les paramètres RSB, et de la au moyen des formules suivantes :

$$E_Y = \frac{1}{2} \times \left(\frac{L_{3M}}{L_{3V}} + \frac{L_{6M}}{L_{6V}}\right)$$

$$E_X = \frac{1}{2} \times \left(\frac{L_{1M}}{L_{1V}} + \frac{L_{5M}}{L_{5V}}\right)$$

$$E_Z = \frac{1}{2} \times \left(\frac{L_{2M}}{L_{2V}} + \frac{L_{4M}}{L_{4V}}\right)$$

formules dans lesquelles l'indice M correspond aux valeurs de longueurs mesurées (figure 10a, 10b, 10c) et l'indice V correspond aux longueurs vraies du fantôme.

Résolution Spatiale R en X, Y et Z

Les résolutions spatiales en X ($R_X$) et Z ($R_Z$) sont calculées à partir par exemple du plan de coupe transverse de la figure 10, après extraction par seuillage dans ce plan de coupe de l'image des inserts 4' et 4" (figure 1 et figure 6) .

Pour le calcul des résolutions spatiales en X ($R_X$) et Z ($R_Z$), tel qu'illustré sur la figure 11, on utilise respectivement deux profils de niveaux de gris $P_X$ (horizontal) et $P_Z$ (vertical) d'un pixel de large passant approximativement par le milieu des pentes qui correspondent aux plans de détection 44a des inserts 4' et 4".

De manière similaire, la résolution spatiale en Y ($R_Y$) est calculée à partir d'un plan de coupe sagittal reconstruit (tel que celui de la figure 10), après extraction par seuillage dans ce plan de coupe de l'image de l'insert 4 et utilisation d'un profil $P_Y$ de niveaux de gris (vertical) d'un pixel de large passant approximativement par le milieu de la pente correspondant au plan de détection 44a de l'insert 4.

En référence à la figure 12, chaque profil $P_X$ , $P_Z$, $P_Y$ brut est lissé avec une courbe spline par exemple, puis la dérivée du profil lissé est calculée.

La résolution en X, Y ou Z est calculée à l'aide de la formule (3) suivante :

$$(3) \quad R_{(X \, ouYouZ)} = \mathrm{FWHM}_{(X \, ouYouZ)} \times \sin(\alpha')$$

dans laquelle FWHM est la largeur à mi-hauteur du profil lissé, interpolé et dérivé.

Lorsque le fantôme est parfaitement aligné avec les trois plans (X,Y), (X,Z) et (Y,Z), l'angle $\alpha'$ de la formule (3) ci-dessus est égal à l'angle $\alpha$ précité des inserts 4, 4' et 4" (soit par exemple 14°).

En revanche, lorsque fantôme 1 n'est pas parfaitement parallèle à l'un des plans (XZ), (X,Y) ou (X,Z) mais est incliné d'un angle $\varepsilon$ (par exemple 1 °) par rapport à ce plan, et lorsque le programme de calcul des paramètres de contrôle qualité comprend l'option de recalage (étape 73 de la figure 7), la valeur d'angle $\alpha'$ utilisée dans la formule (3) est une valeur corrigée ($\alpha' = \alpha - \varepsilon$). L'angle $\varepsilon$, positif ou négatif, est déterminé à partir de la transformation $M_{ref}$ qui a été calculée à l'étape 73.

<u>Profil et largeur de coupe (T)</u>

Le profil de coupe (T) est déterminé à partir d'un plan de coupe transverse de la fente 53 inclinée de l'insert central 5 La méthode utilisée est similaire à celle décrite dans la publication : Bourel Ph, Gibon D., Coste E., Daanen V., Rousseau J., Automatic quality assessment protocol for MRI equipment. Med. Phys. 1999, 26(12) : 2693-2700.

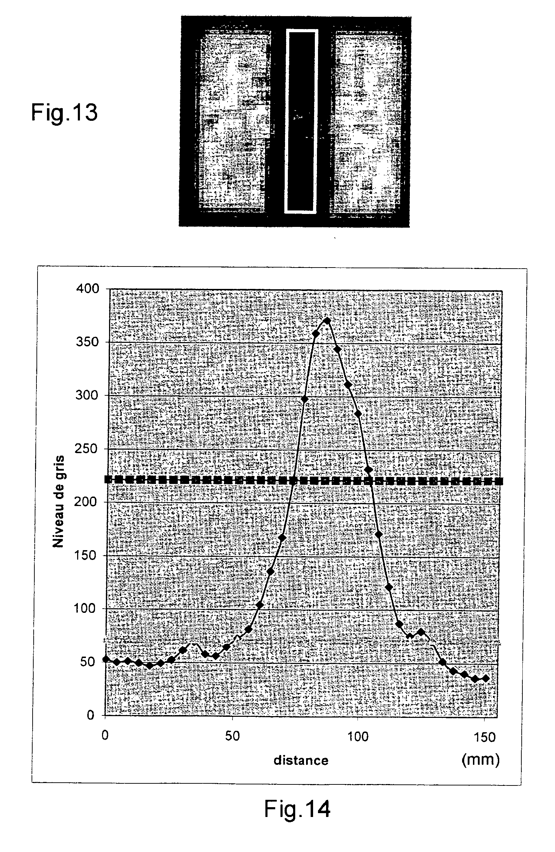

Sur ce plan de coupe transverse passant par l'insert central 5, un profil de niveaux de gris vertical (figure 13), de 8 pixels de large, est défini perpendiculairement à l'espace correspondant à la fente 53 dans le plan de coupe. La courbe brute de niveaux de gris est lissée avec une courbe spline par exemple, afin de s'affranchir du bruit. Un exemple de courbe de niveaux de gris lissée est représenté sur la figure 14.

La largeur de coupe T est calculée sur courbe lissée à l'aide de la formule (4) suivante :

$$(4) \quad T = FWHM \times \tan(\beta')$$

Lorsque le fantôme est parfaitement parallèle au plan coronal (X,Y) l'angle $\beta'$ de la formule (4) ci-dessus est égal à l'angle $\beta$ de la fente 53 de l'insert 5 (soit par exemple 14°).

En revanche, lorsque le fantôme 1 n'est pas parfaitement parallèle à au plan coronal (X,Y) mais est incliné d'un angle $\varepsilon$ (par exemple 1°) par rapport à ce plan, et lorsque le programme de calcul des paramètres de contrôle qualité comprend l'option de recalage (étape 73 de la figure 7), la valeur d'angle $\beta'$ utilisée dans la formule (4) est une valeur corrigée ($\beta' = \beta - \varepsilon$). L'angle $\varepsilon$, positif ou négatif, est déterminé à partir de la transformation $M_{ref}$ qui a été calculée à l'étape 73.

<u>Contraste des volumes chauds et froids</u>

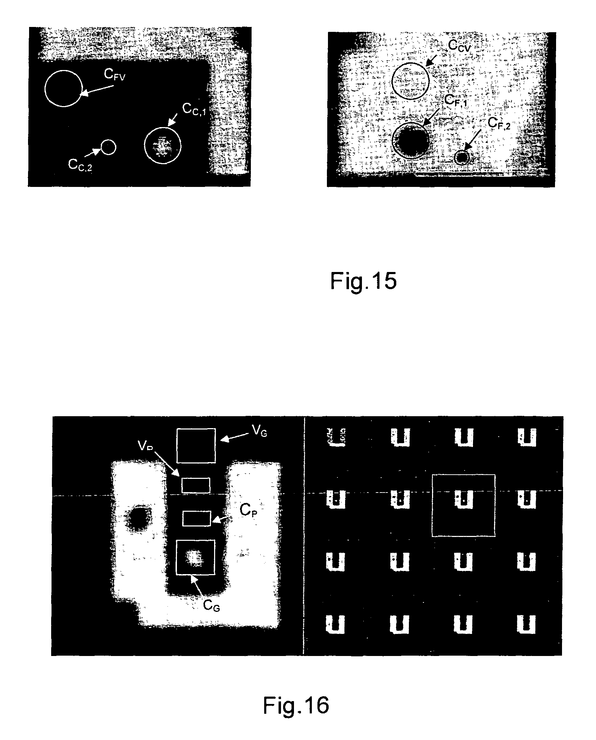

Sur les deux coupes sagittales reconstruites en MPR et passant au mieux par les milieux des objets cylindriques chauds (55,56) et froids (6,7), des régions d'intérêt (ROI) circulaires sont tracées sur chaque cylindre avec un diamètre aussi proche que possible du diamètre vrai du cylindre concerné (soit 10 mm ou 25 mm). Des régions d'intérêt (ROI) sont placées au voisinage des volumes dans le fond chaud et froid (Fig. 15). Les niveaux de gris moyens à l'intérieur de chaque ROI sont mesurés. Les contrastes chauds $K_{C,i}$ et froids $K_{F,i}$ pour chaque volume i (i = 1,2) sont donnés respectivement en pourcentage par :

$$K_{C,i} = 100x \frac{C_{C,i} - C_{FV}}{C_{CV}}$$

$$K_{F,i} = 100x \frac{C_{CV} - C_{F,i}}{C_{CV}}$$

Précision relative de comptage

Dans le cas des imageries tomographiques TEP et TEMP, la précision relative de comptage est mesurée sur les coupes transverses natives. Des régions d'intérêt (ROI) sont positionnées sur les deux cavités cylindriques chaudes 55, 56 (Fig. 16). Les nombres de coups sont additionnés sur le volume entier des cylindres et, après soustraction du bruit de fond, ils sont comparés aux valeurs attendues de comptage. Ces valeurs sont déduites de l'activité spécifique initiale introduite dans le fantôme corrigée de la décroissance radioactive du traceur, et du volume réel du cylindre considéré.

Les précisions relatives de comptage, $R_G$ and $R_P$, sont obtenues, respectivement pour le grand et le petit volume chaud, par :

$$R_G = 100 * \frac{\sum_{i=1}^{N_G}(C_{Gi} - V_{Gi})}{E_G}$$

$$R_P = 100 * \frac{\sum_{i=1}^{N_P}(C_{Pi} - V_{Pi})}{E_P}$$

où $C_{Gi}$ and $C_{Pi}$ correspondent aux coups comptés dans les régions d'intérêt (ROI) positionnées sur le grand et le petit cylindre, respectivement, vues dans la coupe transverse i, $E_G$ and $E_S$ sont les comptages attendus correspondants, $V_{Li}$ and $V_{Si}$ sont les bruits de fond respectifs des régions d'intérêt (ROI) de voisinage, $N_G$ et $N_P$ sont les nombres de coupes transverses dans lesquelles le grand et le petit cylindre sont observés.

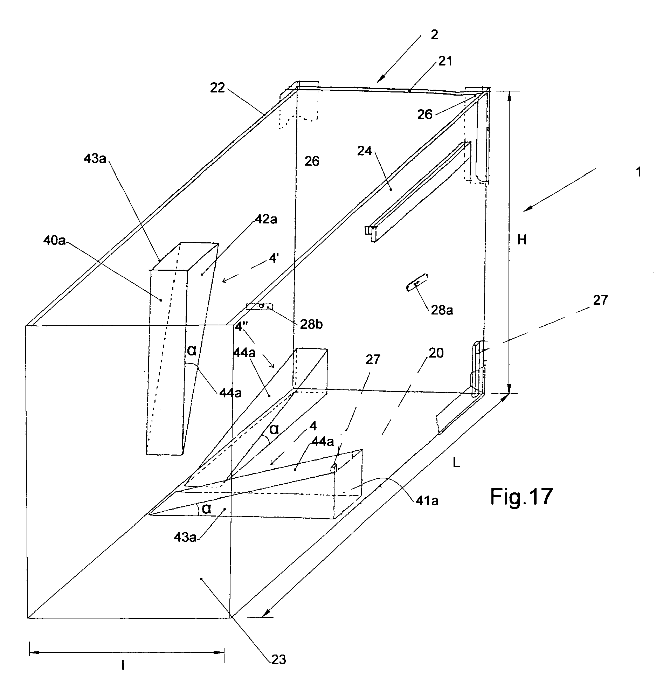

Le fantôme de la figure 1, pourvu d'inserts d'angle fixés dans les angles du bac 2, présente l'avantage d'être compacte et de permettre une acquisition tomographique rapide. L'invention n'est toutefois pas limitée à un fantôme dont le ou les inserts sont fixés dans des angles du bac. A titre d'exemple non limitatif de l'invention, on représenté sur la figure 17, une autre variante de réalisation d'un fantôme conforme à l'invention, pour lesquels les inserts 4 et 4' ne sont pas montés dans des angles du bac. Dans ce cas, s'agissant par exemple de l'insert 4, le plan de calage 40a est positionné contre la paroi de fond 20 du bac ; le plan de calage 43a est parallèle à la paroi latérale 23 (perpendiculaire à la paroi de fond 20), sans être toutefois en contact avec cette paroi 23. Le plan de calage 41a est positionné contre la paroi 24, mais dans une autre variante ce plan de calage 41a pourrait également être distant de la paroi 24. S'agissant de l'insert 4', le plan de calage 43a est positionné contre la paroi latérale 22 du bac, et l'insert 4' est orienté de telle sorte que son plan de calage 40a est parallèle à la paroi latérale 23 (perpendiculaire à la paroi 22), sans être toutefois en contact avec cette paroi.

**Revendications**

1. Fantôme destiné à être utilisé pour le contrôle de qualité en imagerie tomographique, et comportant un bac (2) et au moins deux inserts (4 ou 4' ou 4") fixés à l'intérieur du bac (2), chaque insert comportant un premier plan de calage (40a), un plan de détection (44a), qui est incliné par rapport audit premier plan de calage (40a) d'un angle ($\alpha$) inférieur à 90°, et au moins un deuxième plan de calage (41a) qui est opposé au sommet de l'angle ($\alpha$) et qui est perpendiculaire au premier plan de calage (40a), **caractérisé en ce que** le bac (2) est de forme parallélépipédique, **en ce que** chaque insert (4 ou 4' ou 4") est fixé à l'intérieur du bac (2) d'une part de telle sorte que l'un (40a ou 41a) des plans de calage de l'insert est parallèle à une première paroi du bac (2), et l'autre (41a ou 40a) plan de calage de l'insert est parallèle à une autre paroi du bac (2) perpendiculaire à ladite première paroi, et d'autre part de telle sorte que lesdits deuxièmes plans de calage (41a) des inserts sont perpendiculaires.

**2.** Fantôme selon la revendication 1, **caractérisé en ce qu'**au moins un insert comporte un troisième plan de calage (42a ou 43a), perpendiculaire auxdits premier (40a) et deuxième (41a) plan de calage.

**3.** Fantôme selon la revendication 1 ou 2, **caractérisé en ce qu'**au moins un insert (4 ou 4' ou 4") est fixé à l'intérieur et dans un angle du bac (2), de telle sorte qu'au moins deux plans de calage sont positionnés respectivement contre deux parois adjacentes perpendiculaires du bac (2).

**4.** Fantôme selon la revendication 3, **caractérisé en ce qu'**au moins un insert (4 ou 4") est fixé à l'intérieur et dans un angle du bac (2), de telle sorte que trois plans de calage de l'insert sont positionnés contre respectivement trois parois adjacentes perpendiculaires du bac (2).

**5.** Fantôme selon l'une des revendications 1 à 4, **caractérisé en ce que** un premier (4') et un deuxième (4") inserts sont positionnés l'un par rapport à l'autre de telle sorte qu'au moins un plan de coupe perpendiculaire aux deuxièmes plans de calage (41a) des deux inserts est sécant avec les plans de détection (44a) des deux inserts.

**6.** Fantôme selon la revendication 5, **caractérisé en ce que** ledit premier (4') et ledit deuxième (4") inserts sont positionnés contre la même première paroi latérale (22) du bac (2), **en ce que** le premier insert (4') est positionné contre une deuxième paroi latérale (23) du bac (2) perpendiculaire à la première paroi latérale (22), et **en ce que** le deuxième insert (4") est positionné contre la paroi de fond (20) du bac (2), et de préférence contre une troisième paroi latérale (21) perpendiculaire à la première paroi latérale (22).

**7.** Fantôme selon l'une des revendications 1 à 6, **caractérisé en ce qu'**il comporte un troisième insert comportant un premier plan de calage (40a), un plan de détection (44a), qui est incliné par rapport audit premier plan de calage (40a) d'un angle ($\alpha$) inférieur à 90°, et au moins un deuxième plan de calage (41a) qui est opposé au sommet de l'angle ($\alpha$) et qui est perpendiculaire au premier plan de calage (40a), et **en ce que** l'insert est fixé dans le bac (2) de telle sorte que son deuxième plan de calage (41a) est perpendiculaire aux deuxièmes plans de calage (41a) des deux autres inserts.

**8.** Fantôme selon les revendications 6 et 7, **caractérisée en ce que** ledit troisième insert (4) est fixé dans un angle diamétralement opposé à celui du deuxième insert (4"), en étant positionné au moins contre la paroi de fond (20) du bac (2) et contre la deuxième paroi latérale (23) du bac (2), et de préférence également contre la quatrième paroi latérale (24) du bac parallèle à la première paroi latérale (22).

**9.** Fantôme selon l'une des revendications 1 à 8, **caractérisé en ce que** chaque insert (4, 4' ou 4") présente une section transversale en forme de triangle rectangle.

**10.** Fantôme selon l'une des revendications 1 à 9, **caractérisé en ce que** la valeur de l'angle ($\alpha$) est inférieure à 45°.

**11.** Fantôme selon l'une des revendications 1 à 10, **caractérisé en ce qu'**il comporte un insert central (5), qui est monté à l'intérieur du bac (2), et qui comporte une fente traversante (53) de largeur prédéfinie (e), et inclinée d'un angle ($\beta$) par rapport à un axe vertical perpendiculaire à la paroi de fond (20) du bac (2).

**12.** Fantôme selon la revendication 11, **caractérisé en ce que** la valeur de l'angle ($\beta$) est inférieure à 45°.

**13.** Fantôme selon la revendication 11 ou 12, **caractérisé en ce que** ledit insert central (5) comporte deux cavités cylindriques fermées (55, 56), de diamètres différents, et des canaux (57a, 57b, 57c, 57d) permettant un remplissage par aspiration des deux cavités (55, 56), avec du liquide contenu dans le bac (2).

**14.** Fantôme selon la revendication 11, **caractérisé en ce que** le bac (2) comporte deux rainures (26) perpendiculaires à la paroi de fond (20), et **en ce que** l'insert central (5) est fixé sur une plaque de base (54) apte à être insérée, de manière amovible, dans lesdites rainures (26).

**15.** Fantôme selon l'une des revendications 11 à 14, **caractérisé en ce qu'**il comporte deux inserts supplémentaires (6,7) constitués par deux pièces pleines cylindriques, de même géométrie respectivement que les deux cavités cylindriques (55, 56).

**16.** Fantôme selon l'une des revendications 1 à 15, **caractérisé en ce que** le bac (2) est un parallélépipède rectangle.

**17.** Fantôme selon l'une des revendications 1 à 16, **caractérisé en ce qu'**il comporte un couvercle amovible (3) permettant une fermeture étanche du bac (2).

**18.** Fantôme selon l'une des revendications 1 à 17, **caractérisé en ce qu'**il comporte des moyens (28a, 28b) de contrôle de l'horizontalité de la paroi de fond (20) du bac (2).

**19.** Procédé de préparation du fantôme (1) visé à l'une des revendications 1 à 18, **caractérisé en ce qu'**on remplit le bac (2) avec un liquide détectable dans le mode d'imagerie tomographique.

**20.** Procédé selon la revendication 19, pour la préparation d'un fantôme visé à l'une des revendications 11 à 15, **caractérisé en ce que** l'insert central (5) est monté dans le bac (2) après remplissage dudit bac (2).

**21.** Procédé selon la revendication 19 ou 20, pour la préparation d'un fantôme visé à la revendication 13, **caractérisé en ce qu'**une fois le bac (2) rempli, on remplit par aspiration les deux cavités cylindriques (55, 56) avec du liquide contenu dans le bac (2).

**22.** Procédé selon l'une des revendications 19 à 21, pour la préparation d'un fantôme visé à la revendication 17, **caractérisé en ce qu'**une fois le bac (2) rempli, et le cas échéant une fois les cavités cylindriques (55, 56) de l'insert (5) remplies, on ferme le bac (2) de manière étanche au moyen du couvercle (3).

**23.** Procédé de contrôle de la qualité d'un appareil d'imagerie tomographique au moyen d'un fantôme (1) qui est visé à l'une quelconque des revendications 1 à 18, et dont le bac contient un liquide détectable dans le mode d'imagerie tomographique, **caractérisé en ce qu'**on positionne le fantôme (1) sur la table d'examen de l'appareil d'imagerie tomographique, en l'alignant avec les plans transverse (X,Z), sagittal (Y,Z) et coronal (X,Y) de l'appareil et de telle sorte que :

- la paroi de fond (20) du bac (2) est positionnée parallèlement au plan coronal horizontal (X,Y)
- la première paroi latérale verticale (22) du bac (2) est positionnée parallèlement au plan vertical transverse (X,Z)
- la troisième paroi latérale verticale (23) est positionnée parallèlement au plan vertical sagittal (Y,Z).

**24.** Procédé selon la revendication 23, **caractérisé en ce qu'**une fois le fantôme (1) positionné sur la table d'examen et aligné avec les plans transverse, sagittal et coronal, on réalise les étapes successives suivantes :

(a) tomographie de l'intégralité du fantôme (1) selon des plans de coupe transverse,
(b) de manière facultative, reconstruction par calcul du fantôme (1) dans le plan sagittal et/ou dans le plan coronal, à partir des coupes transverses du fantôme (1) acquises à l'étape (a),
(c) calcul d'un ou plusieurs paramètres de contrôle qualité à partir de coupes transverses du fantôme (1) acquises à l'étape (a), et de manière facultative à partir de coupes sagittales et/ou coronales du fantôme reconstruites à l'étape (b).

**25.** Procédé selon la revendication 24, **caractérisé en ce que** pour le calcul du ou des paramètres de contrôle qualité, on effectue préalablement une étape de recalage du volume du fantôme acquis par tomographie (Volume_CQ) sur un volume de référence (Volume_Ref) jusqu'à obtenir une transformation dans l'espace ($M_{ref}$) entre ces deux volumes, qui répond à un critère de similitude prédéfini.

**26.** Utilisation du fantôme visé à l'une des revendications 1 à 18, pour le contrôle de qualité d'un appareil d'imagerie tomographique, et notamment d'un appareil d'imagerie médicale.

**27.** Ensemble comportant un fantôme (1) visé à l'une des revendications 1 à 18, et un programme de calcul d'un ou plusieurs paramètres de contrôle qualité ($P_i$) d'un appareil d'imagerie tomographique, ledit programme étant enregistré sur un support mémoire ou dans une mémoire, et étant exécutable automatiquement par des moyens de calcul programmables, ledit programme de calcul permettant de calculer automatiquement un ou plusieurs paramètres de contrôle qualité, à partir au moins de coupes transverses qui ont été acquises par tomographie du fantôme (1), au moyen dudit appareil d'imagerie tomographique.

28. Ensemble selon la revendication 27, **caractérisé en ce que** lorsqu'il est exécuté par lesdits moyens de calcul, le programme de calcul permet de reconstruire par calcul les coupes sagittales et/ou coronales du fantôme, à partir des coupes transverses dudit fantôme, et de calculer automatiquement un ou plusieurs paramètres de contrôle qualité à partir desdites coupes sagittales et/ou coronales.

29. Ensemble selon l'une des revendications 27 ou 28, **caractérisé en ce que** lorsqu'il est exécuté par lesdits moyens de calcul, le programme de calcul effectue, préalablement au calcul du ou des paramètres de contrôle qualité, un recalage par calcul du volume du fantôme acquis par tomographie (Volume_CQ) sur un volume de référence (Volume_Ref), jusqu'à obtenir une transformation dans l'espace ($M_{ref}$) entre ces deux volumes, qui répond à un critère de similitude prédéfini.

Fig.1

Fig.3

Fig.4

Fig.5

Fig.2

Fig.6

TOMOGRAPHIE DU FANTÔME
→Coupes transverses (XZ) ———70

Chargement des fichiers
images natives TEP ———71

Reconstruction d'images dans
le plan sagittal et dans le plan
coronal ———72

Recalage de l'acquisition
native sur un volume de
référence
→ $M_{ref}$ ———73

Pour chaque paramètre $P_i$ du
contrôle ———74

i=i+1

Identification de la coupe
d'intérêt pour le calcul de $P_i$ ———75

Calcul du paramètre $P_i$ ———76

Affichage des résultats,
affichage de l'évolution
temporelle des paramètres et
archivage ———77

# Fig.7

anterieur　　　gauche

tête　　　　　　pieds

droite　　　postérieur

Sagittale Trans.

Z

Y

X

Pixel_Trans_₁ (x, z)

(pixel de la vue transverse y=1)

Pixel_Sag_₁ (y, z)

(pixel de la vue sagittale x=1)

Voxel (x, y, z) = Pixel_ Trans _γ(x,z)

(voxel du volume natif à la position (x,y,z))

Fig.8

# Fig.9

Fig.11

Fig.12

(a)  (b)  (c)

Fig.10

Fig.13

Fig.14

Fig.15

Fig.16

Fig.17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0082733 A
- US 7056019 B
- US 20050008126 A
- GB 2155187 A

**Littérature non-brevet citée dans la description**

- **IWELLS III W.M. ; VIOLA P. ; ATSUMI H. ; NAKA-JIMA S. ; KIKINIS R.** Multimodal Volume Registration by Maximisation of Mutual Information. *Medical Image Analysis,* 1997, vol. 1, 35-51
- **PRESS W.H ; FLANNERY B. P ; TEUKOLSKI S. A ; VETTERLING W. R.** Numerical Recipies. C. Cambridge University Press, 1988
- **BOUREL PH ; GIBON D. ; COSTE E. ; DAANEN V. ; ROUSSEAU J.** Automatic quality assessment protocol for MRI equipment. *Med. Phys.,* 1999, vol. 26 (12), 2693-2700